# EUROPEAN PATENT SPECIFICATION

(11) **EP 1 150 686 B1**
(45) Date of publication and mention of the grant of the patent: **12.01.2005**
(21) Application number: 00905514.6
(22) Date of filing: 17.02.2000
(51) Int. Cl.: A61K 31/70, A61K 9/20, A61K 9/28, A61P 31/00

(54) **DIRECTLY COMPRESSIBLE MATRIX FOR CONTROLLED RELEASE OF SINGLE DAILY DOSES OF CLARITHROMYCIN**
DIREKTVERPRESSBARE MATRIX ZUR KONTROLLIERTEN ABGABE VON EINMALTÄGLICHEN DOSEN VON CLARITHROMYCIN
MATRICE DIRECTEMENT COMPRESSIBLE POUR LIBERATION CONTROLEE DE DOSES QUOTIDIENNES UNIQUES DE CLARITHROMYCINE

(30) Priority: 19.02.1999 SI 9900039
(43) Date of publication of application: 07.11.2001
(73) Proprietor: LEK farmacevtska druzba d.d., 1526 Ljubljana (SI)
(72) Inventor: FERCEJ TEMELJOTOV, Darja;, 1000 Ljubljana (SI); MOHAR, Milojka;, 1230 Domzale (SI); SALOBIR, Mateja, 1000 Ljubljana (SI); REBIC, Ljubomira, Barbara;, 1000 Ljubljana (SI); OPRESNIK, Marko, 1000 Ljubljana (SI)
(74) Representative: Müller-Boré & Partner Patentanwälte
(86) International application number: PCT/SI2000/000002
(87) International publication number: WO 2000/048607

(56) References cited:
- WO-A-95/22319

## Description

The present invention belongs to the field of pharmaceutical technology and deals with the macrolide antibiotic clarithromycin.

In the narrow sense the present invention deals with a novel peroral pharmaceutical formulation for controlled release of clarithromycin , enabled by a novel combined matrix consisting of a fatty and a hydrophilic component, whereto a surfactant and a pH modulator can be added when an additional influence on the release profile of the active substance is desired.

Clarithromycin is a slightly alkaline, practically water-insoluble and acid-sensitive macrolide antibiotic. Its solubility decreases with increased temperature and increased pH. A daily dose amounting to 500 mg has to be embedded in a relatively small matrix since a tablet should not be too large to swallow, thus leaving only a relatively narrow space for optimization of biopharmaceutical and physical-technological properties of a formulation. Consequently, in the preparation of a 24-hour tablet we face the problem of a high dose of a poorly soluble active substance and at the same time the need to ensure a repeatable and pH-independent release of clarithromycin according to a specific selected optimal time schedule.

The commercially available peroral formulation of clarithromycin with extended release contains an alginate matrix, which is known for its high dependence of the release of the active substance on the pH, whose preparation technology includes, inter alia, time-consuming and expensive processes of wet granulation, drying and sieving and which frequently also exhibits non-repeatable dissolution profiles between different series and a slowing down of the dissolution due to aging.

Thus, the present invention is based on a need to find a simple, efficient and pH-independent formulation which will repeatably release clarithromycin over 24 hours, thereby minimizing the subjective influences with each single patient. The release rate has to ensure optimal concentrations of the active substance in blood in order to achieve therapeutic effects over a longer time period.

Clarithromycin is a semisynthetic antibiotic formed by methylation of erythromycin on a lactone position of C6. Its synthesis was described in US patents 4,331,803 and 4,672,109. It acts on Gram-positive bacteria and is used clinically due to the wide spectrum of antimicrobial activity. On the market it is present in the form of lacquered tablets, a suspension and extended-release tablets.

Various (per)oral formulations with clarithromycin are also described in the following patent literature:

JP patent 85/163,823 describes an oral drug containing clarithromycin and citric acid increasing the absorption of the antibiotic in the digestive tract, disintegrants, carriers and lubricants.

Withdrawn EP patent application No. 277,042 describes an oral pharmaceutical formulation with improved taste, with a coating made of special polymers (especially polyvinylacetal diethylaminoacetate - AEA) soluble in gastric juice and with average particle diameters under 60 µm.

US patent 4,808,411 describes a formulation with erythromycin or its derivatives and a carbomer, optionally in the form of particles of an ionic complex, coated with a polymer, whereat the particles can be suspended in a liquid carrier.

JP patent 89/42,625 describes the preparation of film-coated microgranules of a drug with sustained action, which, in addition to clarithromycin, also contain AEA and water.

Withdrawn EP patent application No. 302,370 and WO patent application No. 90/08,537 describe improved (per)oral formulations (oily solution, suspension, emulsion) of erythromycin and its derivatives for filling into soft gelatin capsules with N-methyl-pyrrolidone.

EP patent 420,992 describes a process for producing a taste-masked oral formulation comprising spraying a suspension of a drug into a cold aqueous solution of AEA.

US patent 5,017,383 describes a method of producing a finely coated pharmaceutical formulation comprising mixing frozen particles of a liquid medium with a drug and a coating in the form of a fine powder adhering to the surface of the particles.

US patents 5,599,556 and 5,609,909 describe the taste-masking of encapsulated clarithromycin particles with prolamine coatings prior to the preparation of a suspension.

WO patent application No. 96/34,628 describes a formulation with masked taste for oral administration (especially a dry syrup), containing an unpleasantly tasting drug, a high polymer soluble in the stomach (preferably AEA or Eudragit E) and a monoglyceride having a low melting point (preferably glyceryl monostearate) in a stable β-crystal form (transfer from meta stable α-form by means of shaking at an elevated temperature) and a method for masking the taste.

WO patent application No. 97/16,174 describes a process for water-granulation of a macrolide antibiotic with a carbomer (acrylic polymer).

US patent 5,705,190 describes a solid oral pharmaceutical formulation with controlled release containing a drug poorly soluble in water, a water-soluble alginate salt, a complex salt of alginic acid with a metal cation and an organic carboxylic acid facilitating the dissolution of the drug.

US patent 5,707,646 describes a formulation for oral administration (especially dry syrup) containing an unpleasantly tasting drug, a functional polymer (preferably AEA and/or Eudragit E) in a substance having a melting point at 40-120°C, a sugar alcohol (e.g. sorbitol) and a basic oxide (preferably MgO).

WO patent application No. 98/46,239 describes a pharmaceutical formulation with extended action containing an erythromycin derivative and a hydrophilic water-soluble polymer, showing, at oral administration, an improved taste profile and fewer gastrointestinal side effects in comparison to the usual formulation, and the preparation technology comprises, inter alia, processes of wet granulation, drying, sieving and milling.

WO patent application 95/22,319 discloses a process having improved throughput for preparing fine particle pharmaceutical formulations which exhibit improved uniformity of particle size and performance characteristics such as drug release.

Thus, in patent and other literature from this field there can be found numerous publications describing the composition and the preparation of various formulations with clarithromycin, but no literature source has been found that would describe such a simple process for the preparation of a clarithromycin formulation with controlled release, enabling a selected and pH-independent release profile of the active substance over 24 hours.

The object of the invention is a novel matrix for a controlled release of clarithromycin , which contains a mixture of a fatty, water-insoluble component being the main carrier of sustained release, and of a hydrophilic component that in an aqueous medium swells, gels or thickens and thereby forms a viscous layer through which the solubilized, dissolved active substance diffuses, thus influencing the structure and consistency of the whole matrix.

To this basic matrix there may be added a wetting agent, a surfactant which softens the matrix and binds together both types of components and contributes to an easier solubilization of the active substance. The result is a matrix system which by means of a mixed mechanism of tablet erosion and diffusion releases the solubilized and/or dissolved active substance through the viscous layer.

In order to additionally influence the release profile of the active substance, to the matrix there may optionally be added a pH modulator, which is an alkaline substance, e.g. a phosphate buffer, influencing the portion of the released active substance in the stomach in relation to the intestine or decreasing the influence of the current level of acidity in any particular part of the digestive tract.

Among fatty components to be used in the embodiments of the present invention, the suitable ones are triglycerides of higher saturated fatty acids such as palmitic acid, stearic acid and behenic acid, preferably glyceryl behenate, hydrogenated oils (e.g. vegetable oils or castor oil), carnauba wax and similar. The content of the fatty component of the matrix amounts to about 10-36 % of the mass of a tablet.

Glyceryl behenate is a lipid substance, most frequently used as a lubricant, with an additional favourable property that in higher concentrations it sustains the release of active substances. Chemically, it is a mixture of glyceryl esters of behenic/docozanoic acid (C₂₂) with a low content of mono-behenate and a melting point of 69-74°C.

As the hydrophilic component there are selected substances which increase the viscosity of the micro-environment, are suspendable, stabilize the current viscous layer and may also have the ability to soften the fatty components of the matrix.

Suitable substances are alkyl-substituted cellulose ethers, preferably hydroxypropyl methylcellulose (HPMC), more preferably HPMC with low viscosity, fatty alcohols (e.g. cetyl, stearyl, cetostearyl alcohols), polysaccharides (e.g. xanthan gum, guar gum, acacia), adsorbents with a large specific surface (e.g. Mg-Al-silicates) and the like. The content of the hydrophilic component of the matrix amounts to about 5-18 % of the mass of a tablet.

HPMC is a cellulose ether, usually used for increasing the viscosity of the environment, but simultaneously it also influences the release rate of active substances. Used were low-viscous types of HPMC having viscosities (nominally for a 2 % aqueous solution at 20°C) up to about 40 cP and Mₙ up to about 20000 (determined by the method of osmotic pressures).

It has been found that by combining glyceryl behenate and HPMC there was obtained an exceptionally effective matrix for sustaining and controlling the release of the active substance, since in contact with an aqueous medium (also in the stomach) it swells and thereby loosens the lipid (glyceryl behenate) structure and makes possible a release of clarithromycin by diffusion through the viscous layer at a simultaneous erosion of the matrix i.e the tablet. The ratio between the lipophilic and the hydrophilic components may be between 2 : 1 and 10 : 1.

Among surfactants, anionic ones e.g. sodium docusate, sodium lauryl sulfate, and nonionic ones can be used. The content of the surfactant amounts to about 0.5-3% of the mass of a tablet.

Sodium docusate is an anionic surfactant which in the given combination contributes to a more uniform wetting of the lipid structure and thereby facilitates the hydratation and the swelling of the matrix and makes possible a repeatable diffusion of clarithromycin from the matrix.

Tablets with clarithromycin can also be lacquered, e.g. with a suspension based on a mixture of HPMC and hydroxypropyl cellulose (HPC), according to a conventional process or the release profile of clarithromycin is modulated by the application of an acid-resistant coating such as HPMC-phthalate.

An excellent property of the formulation of the present invention in comparison to prior art is a simple preparation technology since all ingredients are, at room temperature, just homogeneously mixed together, sieved and directly compressed into tablets, therefore no water or any other solvents are necessary.

An important technological advantage of the formulation of the present invention is also the fact that there is no need for additional lubricants, since glyceryl behenate itself, as the carrier of release control, has good lubricating properties.

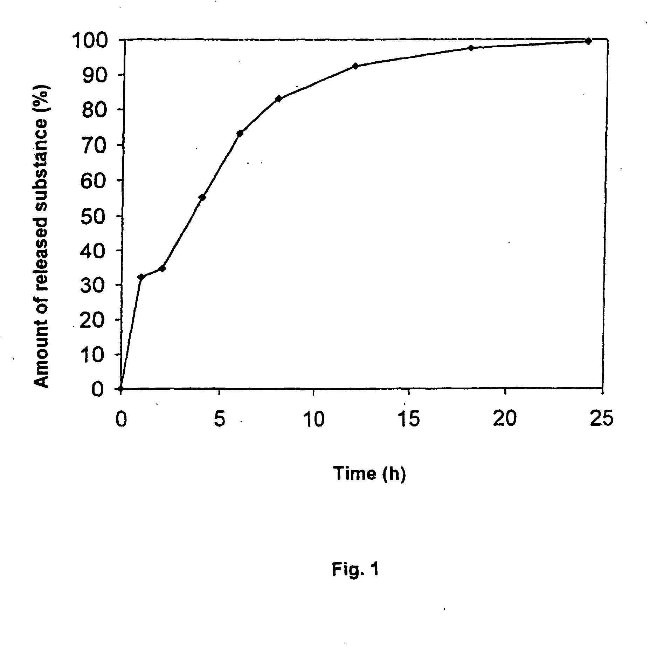

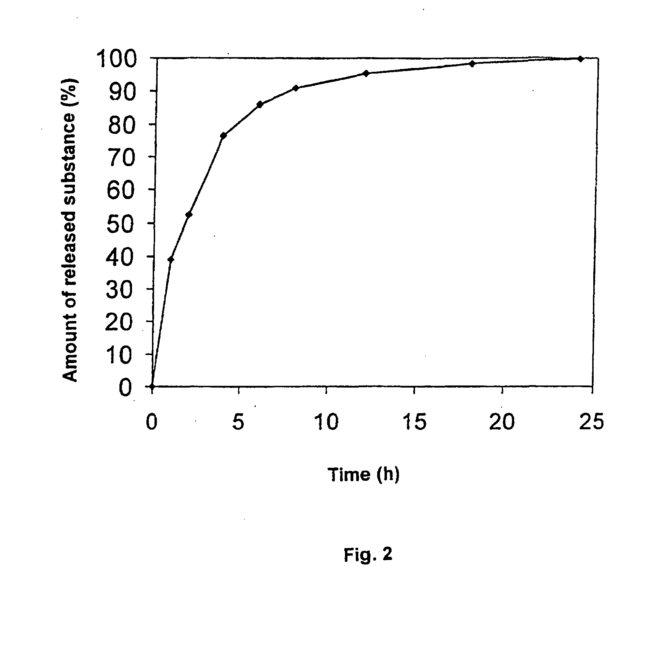

Dissolution rates of clarithromycin *in vitro* from two matrix samples with controlled release over 24 hours were measured at the temperature of 37°C, during the first hour at pH = 3.0 and during the subsequent 23 hours at pH 6.8. They are shown in Figs. 1 and 2.

### Example 1

| | | |
|---|---|---|
| Composition of one tablet | clarithromycin | 500 mg |
| | glyceryl behenate (Compritol 888) | 350 mg |
| | HPMC (E50-LV P) | 150 mg |
| | lactose | 150 mg |
| Dissolution test | | |

Dissolution profile is shown in Fig. 1.

### Example 2

The composition was the same as in Example 1 only that instead of 34.5 g of lactose the same quantity of Na-docusate was used.

### Example 3

| | | |
|---|---|---|
| Composition of one tablet | clarithromycin | 500 mg |
| | glyceryl behenate (Compritol 888) | 350 mg |
| | HPMC (E50-LV P) | 150 mg |
| | lactose | 131.905 mg |
| | NaH₂PO₄ | 49.538 mg |
| | Na₂HPO₄ | 2.607 mg |
| | Na-docusate | 5.95 mg |
| Dissolution test | | |

Dissolution profile is shown in Fig. 2.

### Example 4

| | | |
|---|---|---|
| Composition of one tablet | clarithromycin | 500 mg |
| | glyceryl behenate (Compritol 888) | 350 mg |
| | HPMC (E15-LV P) | 150 mg |
| | polyvinyl pyrrolidone (K 25) | 60 mg |
| | microcrystalline cellulose | 40 mg |
| | stearic acid | 15 mg |
| | SiO₂ (Aerosil 200) | 5 mg |
| | talc | 5 mg |
| | Ca-stearate | 25 mg |

### Example 5

| | | |
|---|---|---|
| Composition of one tablet | clarithromycin | 500 mg |
| | glyceryl behenate (Compritol 888) | 350 mg |
| | HPMC (E50-LV P) | 150 mg |
| | polyvinyl pyrrolidone (K 25) | 60 mg |
| | microcrystalline cellulose | 40 mg |
| | stearic acid | 15 mg |
| | SiO₂ (Aerosil 200) | 5 mg |
| | talc | 5 mg |
| | Ca-stearate | 25 mg |

### Example 6

The composition of a tablet was the same as in Examples 1-5 only that on the tablet also an acid-resistant coating with the following composition was applied:

| | |
|---|---|
| HPMC-phthalate | 28.75 mg |
| triethyl citrate | 2.875 mg |
| yellow pigment (Fe-oxide) | 0.822 mg |
| TiO₂ | 0.514 mg |
| talc | 4.039 mg |

## Claims

1. A pharmaceutical controlled release formulation for peroral single daily application, **characterized in that** it comprises clarithromycin and a mixture of a fatty and a hydrophilic component.

2. The pharmaceutical controlled release formulation according to claim 1, **characterized in that** in addition to the given components it comprises a surfactant.

3. The pharmaceutical controlled release formulation according to claim 1 or 2, **characterized in that** in addition to the given components it comprises a pH modulator.

4. The pharmaceutical controlled release formulation according to anyone of claims 1 to 3, **characterized in that** in addition to the given components it comprises other pharmaceutically acceptable additives.

5. The pharmaceutical controlled release formulation according to anyone of claims 1 to 4, **characterized in that** the fatty component comprises glyceryl behenate.

6. The pharmaceutical controlled release formulation according to anyone of claims 1 to 5, **characterized in that** the hydrophylic component comprises hydroxypropyl methylcellulose having viscosities up to 40 cP and Mₙ up to 20 000.

7. The pharmaceutical controlled release formulation according to claim 6, **characterized in that** hydroxypropyl methylcellulose has a viscosity of about 15 cP.

8. The pharmaceutical controlled release formulation according to anyone of claims 2 to 7, **characterized in that** the surfactant comprises sodium docusate.

9. The pharmaceutical controlled release formulation according to anyone of claims 3 to 8, **characterized in that** the pH modulator comprises a phosphate buffer.

10. A pharmaceutical controlled release formulation according to anyone of claims 1 to 9, **characterized in that** it is in the form of a tablet.

11. A pharmaceutical controlled release formulation according to claim 10, **characterized in that** the tablet is lacquered.

12. A pharmaceutical controlled release formulation according to claim 10, charcterized in that on the tablet an acid-resistant coating is applied.

13. A process for the preparation of a pharmaceutical controlled release formulation according to anyone of claims 1 to 12, **characterized in that** it comprises homogenous mixing, sieving and direct compressing into tablets without the use of solvents.

14. A pharmaceutical controlled release formulation for peroral single daily application, **characterized in that** it is prepared according to the process according to claim 13.

15. A pharmaceutical controlled release formulation according to claims 1 to 12 for use in the treatment and prophylaxis of bacterial infections.

## Patentansprüche

1. Eine pharmazeutische Formulierung mit kontrollierter Abgabe zur peroralen einmaltäglichen Verbareichung, **dadurch gekennzeichnet, dass** sie Clarithromycin und eine Mischung einer Fettkomponente und einer hydrophilen Komponente enthält.

2. Die pharmazeutische Formulierung mit kontrollierter Abgabe gemäß Anspruch 1, **dadurch gekennzeichnet, dass** sie zusätzlich zu den angegebenen Komponenten ein oberflächenaktives Mittel enthält.

3. Die pharmazeutische Formulierung mit kontrollierter Abgabe gemäß Anspruch 1 oder 2, **dadurch gekennzeichnet, dass** sie zusätzlich zu den angegebenen Komponenten einen pH-Modulator enthält.

4. Die pharmazeutische Formulierung mit kontrollierter Abgabe gemäß irgendeinem der Ansprüche 1 bis 3, **dadurch gekennzeichnet, dass** sie zusätzlich zu den angegebenen Komponenten andere pharmazeutisch annehmbare Zusatzmittel enthält.

5. Die pharmazeutische Formulierung mit kontrollierter Abgabe gemäß irgendeinem der Ansprüche 1 bis 4, **dadurch gekennzeichnet, dass** die Fettkomponente Glyceryl-Behenat umfasst.

6. Die pharmazeutische Formulierung mit kontrollierter Abgabe gemäß irgendeinem der Ansprüche 1 bis 5, **dadurch gekennzeichnet, dass** die hydrophile Komponente Hydroxypropylmethylcellulose mit Viskositäten bis 40 cP und Mₙ bis 20 000 umfasst.

7. Die pharmazeutische Formulierung mit kontrollierter Abgabe gemäß Anspruch 6, **dadurch gekennzeichnet, daß** Hydroxypropylmethylcellulose eine Viskosität von etwa 15 cP hat.

8. Die pharmazeutische Formulierung mit kontrollierter Abgabe gemäß irgendeinem der Ansprüche 2 bis 7, **dadurch gekennzeichnet, dass** das oberflächenaktive Mittel Docusat-Natrium umfasst.

9. Die pharmazeutische Formulierung mit kontrollierter Abgabe gemäß irgendeinem der Ansprüche 3 bis 8, **dadurch gekennzeichnet, dass** der pH-Modulator einen Phosphatpuffer umfasst.

10. Eine pharmazeutische Formulierung mit kontrollierter Abgabe gemäß irgendeinem der Ansprüche 1 bis 9, **dadurch gekennzeichnet**, das sie in der Form einer Tablette ist.

11. Eine pharmazeutische Formulierung mit kontrollierter Abgabe gemäß Anspruch 10, **dadurch gekennzeichnet, dass** die Tablette lackiert ist.

12. Eine pharmazeutische Formulierung mit kontrollierter Abgabe gemäß Anspruch 10, **dadurch gekennzeichnet, dass** auf die Tablette ein saürebeständiger Überzug aufgebracht ist.

13. Ein Verfahren zur Herstellung einer pharmazeutischen Formulierung mit kontrollierter Abgabe gemäß irgendeinem der Ansprüche 1 bis 12, **dadurch gekennzeichnet, dass** es homogenes Vermischen, Sieben und direktes Zusammenpressen zu Tabletten ohne Verwendung von Lösungsmitteln umfasst.

14. Eine pharmazeutische Formulierung mit kontrollierter Abgabe zur peroralen einmaltäglichen Verabreichung, **dadurch gekennzeichnet, dass** sie gemäß dem Verfahren nach Anspruch 13 bereitet wird.

15. Eine pharmazeutische Formulierung gemäß Ansprüchen 1 bis 12 zur Verwendung in der Behandlung und Prophylaxe von bakteriellen Infektionen.

## Revendications

1. Formulation pharmaceutique à libération contrôlée pour une application pérorale quotidienne unique, **caractérisée en ce qu'**elle comprend de la clarithromycine et un mélange d'un composant gras et d'un composant hydrophile.

2. Formulation pharmaceutique à libération contrôlée selon la revendication 1, **caractérisée en ce que**, outre les composants donnés, elle comprend un agent tension-actif.

3. Formulation pharmaceutique à libération contrôlée selon la revendication 1 ou 2, **caractérisée en ce que**, outre les composants donnés, elle comprend un modulateur de pH.

4. Formulation pharmaceutique à libération contrôlée selon l'une quelconque des revendications 1 à 3, **caractérisée en ce que**, outre les composants donnés, elle comprend d'autres additifs acceptables d'un point de vue pharmaceutique.

5. Formulation pharmaceutique à libération contrôlée selon l'une quelconque des revendications 1 à 4, **caractérisée en ce que** le composant gras comprend du béhénate de glycérol.

6. Formulation pharmaceutique à libération contrôlée selon l'une quelconque des revendications 1 à 5, **caractérisée en ce que** le composant hydrophile comprend de l'hydroxypropylméthylcellulose avant des viscosités allant jusqu'à 40 cP et une masse moléculaire en nombre (Mn) allant jusqu'à 20 000.

7. Formulation pharmaceutique à libération contrôlée selon la revendication 6, **caractérisée en ce que** l'hydroxypropylméthylcellulose a une viscosité d'environ 15 cP.

8. Formulation pharmaceutique à libération contrôlée selon l'une quelconque des revendications 2 à 7, **caractérisée en ce que** l'agent tensio-actif comprend du docusate de sodium.

9. Formulation pharmaceutique à libération contrôlée selon l'une quelconque des revendications 3 à 8, **caractérisée en ce que** le modulateur de pH comprend un tampon phosphate.

10. Formulation pharmaceutique à libération contrôlée selon l'une quelconque des revendications 1 à 9, **caractérisée en ce qu'**elle se présente sous la forme d'un comprimé.

11. Formulation pharmaceutique à libération contrôlée selon la revendication 10, **caractérisée en ce que** le comprimé est laqué.

12. Formulation pharmaceutique à libération contrôlée selon la revendication 10, **caractérisée en ce qu'**on applique sur le comprimé un revêtement résistant à l'acide.

13. Procédé pour la préparation d'une formulation pharmaceutique à libération contrôlée selon l'une quelconque des revendications 1 à 12, **caractérisé en ce qu'**il comprend le mélange homogène, le tamisage et la compression directe en comprimés sans utiliser de solvant.

14. Formulation pharmaceutique à libération contrôlée pour une application pérorale quotidienne unique, **caractérisée en ce qu'**elle est préparée en utilisant le procédé selon la revendication 13.

15. Formulation pharmaceutique à libération contrôlée selon l'une quelconque des revendications 1 à 12, destinée à être utilisée pour le traitement et la prophylaxie des infections microbiennes.
